# EUROPEAN PATENT SPECIFICATION

(11) **EP 0 759 991 B1**
(45) Date of publication and mention of the grant of the patent: **21.04.1999**
(21) Application number: 95919498.6
(22) Date of filing: 18.05.1995
(51) Int. Cl.: C12N 15/52, C12N 9/00, C12Q 1/68

(54) **GENES INVOLVED IN THE BIOSYNTHETIC PATHWAY OF CARBAPENEM**
IN DER BIOSYNTHESE VON CARBAPENEN INVOLVIERTE GENE
GENES IMPLIQUES DANS LA VOIE BIOSYNTHETIQUE D'UN CARBAPENEM

(30) Priority: 20.05.1994 GB 9410142
(43) Date of publication of application: 05.03.1997
(73) Proprietor: UNIVERSITY OF WARWICK, Coventry, Warwickshire CV4 7AL (GB); THE UNIVERSITY OF NOTTINGHAM, Nottingham NG7 2RD (GB)
(72) Inventor: SALMOND, G.P.C., Cambridge, Cambridgeshire (GB); McGOWAN, Simon James, Tile Hill, Coventry CV4 9FU (GB); SEBAIHIA, Mohammed, Styvechale, Coventry CV3 5LX (GB); COX, Anthony Richard John, Chadwick End, Solihull B93 0BP (GB); HOLDEN, Matthew Thomas Geoffrey, Coventry CV4 7AL (GB); PORTER, Lauren Elizabeth, Tile Hill, Coventry CV4 9ST (GB); BYCROFT, Barrie Walsham, Beeston, Nottingham NG9 2FR (GB); WILLIAMS, Paul, Wollaton, Nottingham NG8 1HR (GB); STEWART, Gordon Sidney Anderson Birnie, Leicester LE11 0QL (GB)
(74) Representative: Bannerman, David Gardner
(86) International application number: PCT/GB95/01125
(87) International publication number: WO 95/32294

(56) References cited:
- WO-A-94/28135
- Patent Abstracts of Japan JP1043191, publication date 15.02.89, Sanraku Inc.
- THE BIOCHEMICAL JOURNAL, vol. 288, no. 3, 15 December 1992 pages 997-1004, BAINTON, N.J. ET AL. 'N-(3-Oxohexanoyl)-L-homoserine lactone regulates carbapenem antibiotic production in Erwinia carotovora' cited in the application
- MOLECULAR MICROBIOLOGY, vol. 10, no. 3, 1993 pages 511-520, SWIFT, S. ET AL. 'A novel strategy for the isolation of luxI homologues: evidence for the widespread distribution of a LuxR:LuxI superfamily in enteric bacteria'
- MICROBIOLOGY, vol. 141, no. 3, March 1995 pages 541-550, MCGOWAN, S. ET AL. 'Carbapenem antibiotic production in Erwinia carotovora is regulated by carR, a homologue of the LuxR transcriptional activator'

## Description

The present invention relates to the bacterial gene expression of carbapenem antibiotics.

The carbapenem antibiotics constitute a diverse group of β-lactam antibiotics characterised by potent anti-bacterial and 8-lactamase - resistant activity. More than forty different carbapenems are known, most of which are produced by the actinomycetes, particularly Streptomyces spp (Ratcliffe and Albers-Schonberg, 1982; Brown 1984; Williamson 1986; all cited in Baiton et al 1992).

Carbapenems have been isolated from the Gram-negative bacterium Serratia marcescens and Erwinia carotovora by Parker et al. (1982) and in Azospirillum spp UK 1521 by Kintaka et al. (1985); all cited in Bainton et al 1992.

Bainton et al (1992) have recently shown that carbapenem biosynthesis is regulated by the regulatory factor N-(3-oxohexanoyl)-L-homoserine lactone (known as HSL or OHHL). This compound was previously only known for its role in auto-induction of bioluminescence in the marine bacterium Vibrio fischeri. OHHL is also structurally related to the A- and I- factors which are known to regulate production of antibiotics in some Streptomyces species.

JP-1043191 discloses DNA fragments from Streptomyces species which, on introduction to carbapenem non-producing mutants of Streptomyces fulvobiridis. stimulate antibiofic production. However, the precise nature or function of the fragments is not disclosed in the document.

In order to examine the biosynthetic and regulatory mechanism involved in the production of the β-lactam antibiotic 1-carbapen-2-em-3-carboxylic acid by Erwinia carotovora, blocked mutants were obtained with a carbapenem non-producing phenotype (Car⁻) as described by Bainton et al (1992). These mutants fell into two distinct groups: group 1 mutants secreted a low molecular mass diffusible factor which restored carbapenem biosynthesis in group 2 mutants, but not vice versa. This factor was shown to be OHHL. Class 1 mutants produced OHHL and were thus thought to be defective in carbapenem biosynthetic genes.

In order to study class 1 mutants a chromosomal DNA cosmid library of Erwinia carotovora strain ATCC 39048 was constructed in Escherichia coli. The cosmids produced were used in standard complementation studies to find a sequence which could restore the carbapenem antibiotic production in the class 1 mutants.

One cosmid (cWU142) was presumed to contain the carbapenem biosynthetic genes. Restriction fragments of this cosmid were sub-cloned and a 3.8 Kb EcoRI fragment was found to complement 7 out of 8 class 1 mutants. This fragment was sequenced and shown to comprise 2 Kb of cosmid DNA and 1.8 Kb of Erwinia DNA. This was extremely unexpected because known antibiotic biosynthetic gene sequences are much longer than 1.8 Kb.

The 1.8 Kb gene sequence was found to encode CarR, a homologue of the LuxR regulatory protein from the Lux operon system associated with the bioluminescence phenotype of the marine bacterium V. fischeri.

In V. fischeri OHHL synthesis is coded for by the gene luxI. In Erwinia carotovora, OHHL synthesis is encoded by a luxI homologue which has been named carI.

By analogy with the V. fischeri Lux system, the inventors postulated that when OHHL is made, it binds to CarR which can then act as a transcriptional activator of the carbapenem biosynthetic genes. Thus, the inventors reasoned that 7 out of 8 of the class 1 mutants are not, as expected, defective in genes required for synthesis of carbapenem, but in a gene encoding a regulatory protein, CarR, needed to switch on the carbapenem biosynthesis genes. Without the carR gene product the carbapenem biosynthetic genes are not expressed, that is, they remain silent or tryptic.

Our co-pending application GB-9311641.6 relates to the carR gene product and to DNA sequences encoding it.

We therefore used direct cosmid complementation of a strain of class 1 mutants (strain PNP 14) to clone a complementing cosmid (cWU142). We presumed that this would carry all the biosynthetic genes. When cWU142 was transferred into other class 1 mutants it complemented all of them. cWU142 did not complement any class 2 mutants (this is because the carI gene is physically separate from the biosynthetic genes). We showed that the cWU142 cosmid carries the gene (carR) encoding the LuxR homologue. The CarR protein acts as a positive activator of the car biosynthetic genes and functions in trans. This carR gene is capable of activation of carbapenem antibiotic production in several other Erwinia strains (approx. 18-20% of random strains). One of the class 1 mutants (PNP21) was not complemented by the carR gene alone and was presumed to be a real biosynthetic mutant. cWU142 complemented this mutant, implying that the car biosynthetic genes were indeed on the cosmid. This was confirmed by the observation that, in E. coli, cWU142 could encode carbapenem synthesis simply by the exogenous addition of chemically-synthesised OHHL. It seems that the car biosynthetic genes are not expressed because the CarR protein requires activation by the OHHL although the requirement for OHHL can be over-ridden by producing CarR in high dosage e.g. by providing the carR gene in trans on a multicopy plasmid. In Erwinia carotovora there is endogenous OHHL so the car genes are switched on.

Thus all genes for antibiotic production and one for regulation (carR) were on cWU142, permitting the carbapenem antibiotic biosynthetic pathway to be reconstituted in a heterologous organism (in this case E. coli).

The cWU142 cosmid was subcloned to produce smaller plasmids which still express the antibiotic. Plasmid pSMG12 encoded antibiotic synthesis in E. coli in the presence of exogenously added OHHL. Plasmid pSMG13 was engineered to contain the carI gene in addition to the genes found in pSMG12. Plasmid pSMG13 encoded carbapenem production without the need for exogenously added OHHL. This strain makes OHHL (from the carI gene product) and apparently acts with CarR to transcriptionally activate the carbapenem biosynthetic genes. Further subcloning of the pSMG13 plasmid has shown that the cluster of 9 genes carR,carA,carB,carC,carD,carE,carF,carG,carH is sufficient to express carbapenem in E.coli if OHHL is added. OHHL is not required if the carI gene is also present and functional. All of the Erwinia DNA in the pSMG12/pSMG13 plasmid has been sequenced to define the genes carR to carH.

According to one aspect of this invention we provide, either separately or in combination, DNA consisting of the genes carA,carB,carC,carD,carE,carF,carG,carH.

Preferably, we provide DNA consisting of at least the CarA, CarB and CarC genes. More preferably we provide DNA consisting of the CarA, CarB, CarC, CarD and CarE genes.

Also included within the invention are DNA capable of hybridising to any of the DNAs defined above and capable of functioning as such DNAs in the carbapenem biosynthetic pathway, and DNAs which are equivalent by virtue of the degeneracy of the genetic code.

The CarF and CarG genes are now known to be involved in conferring resistance to the antibiotic produced by the organism.

The DNA may also include either or both of the regulatory genes carI and carR.

The DNA according to the invention may comprise genes of Erwinia carotovora, or DNA capable of hybridising to any of said genes and capable of functioning as such genes in the biosynthetic pathway of a carbapenem.

Such hybridising DNA may comprise native genes from other carbapenem-synthesising organisms or partially or wholly synthetic DNA.

We also provide DNA which is equivalent to any DNA mentioned above by virtue of the degeneracy of the genetic code.

Portions of the Erwinia carotovora genes or corresponding genes from other organisms are included provided that such portions can function in carbapenem synthesis.

We also provide a polypeptide coded for by DNA as defined hereinabove.

We further provide a recombinant DNA molecule including any DNA as defined hereinabove.

We also provide a recombinant DNA molecule, including the cluster of genes carA,carB,carC,carD, carE,carF,carG*,*carH. Such a recombinant DNA molecule may also contain either or both of the regulatory genes carR and carI, or may be placed under the control of a heterologous regulated promoter. The recombinant DNA molecule is preferably an expression vector capable of expressing a carbapenem coded for by said gene cluster.

In such a recombinant DNA molecule, any of the above-mentioned genes may be replaced by DNA capable of hybridizing thereto and which is itself capable of functioning in the carbapenem biosynthetic pathway.

Any of the above-mentioned genes may be replaced by DNA which is equivalent to it by virtue of the degeneracy of the genetic code.

Examples of recombinant DNA molecules as mentioned above are the cosmid cWU142 and plasmids pSMG12 and pSMG13.

We also provide a method of identifying further DNA coding for production of a carbapenem by using a DNA sequence as defined hereinabove, and preferably at least a substantial part of one or more of the genes carR,carA,carB,carC,carD, carE,carF,carG,carH as a hybridization probe, incorporating any further DNA so identified into an expression vector, transforming a host with the resulting recombinant expression vector, expressing the polypeptide coded for by said further DNA, and testing said polypeptide for carbapenem biosynthetic activity.

The known carbapenem genes or portions thereof may thus be used to search for homologues in other bacteria as well as other organisms such as fungi. The procedure is particularly appropriate for searching for carbapenem gene homologues in streptomycetes producing Thienamycin and other carbapenems. The portions of the above-mentioned genes to be used as hybridization probes may if necessary be modified by replacing codons by codons coding for the same amino acids but more appropriate to the organism under investigation.

The Erwinia carotovora strain used throughout was Erwinia carotovora subspecies carotovora (ATCC39048).

An internal StuI fragment from the car cluster of the strain of Erwinia cartovora described above (containing DNA internal to carA through to a site internal to carG) was used to probe chromosomal DNA from various other Erwinia carotovora strains. Of 16 strains probed, 10 yielded positive hybridisation signals. The StuI probe was also used to probe DNA from other Erwinia carotovora subspecies, and bacteria of other genera, namely Serratia marcescens and Streptomyces cattleya. Positive signals were seen with Erwinia carotovora subspecies atroseptica (strain SCRI39), Erwinia carotovora subspecies betavasculorum (strain SCRI479), Serratia marcescens (ATCC39006) and Streptomyces cattleya (strain 8057). We therefore believe that those strains showing positive hydridisation signals carry genes homologous to the car genes of our original strain (ATCC39048). In fact we have established that Serratia marcescens (ATCC39006) produces exactly the same antibiotic as the original Erwinia carotovora strain. We have determined the sequence of the complete Car cluster from Serratia marcescens (ATCC39006) and all of the genes are highly homologous to the corresponding genes of Erwinia carotovora (ATCC39048).

We have also performed PCR amplificiation of carC homologues in Streptomyces cattleya . The rationale for this is that antibiotic synthesis (and resistance) genes are usually clustered in bacteria and so cloning part of any one gene in the Streptomyces cluster then provides a "probe" with which to clone the rest of the cluster.

In addition to knowing the total car cluster sequence from Erwinia carotovora, we also cloned and sequenced the homologous sequences from Serratia marcescens (strain ATCC39006). As with the Erwinia cluster, the equivalent car cluster from Serratia also encodes carbapenem synthesis when cloned in E.coli. Two 18mer oligonucleotide primers were designed based on totally conserved amino acids in the predicted protein sequence of the Erwinia carotovora and Serratia marcescens carC genes (strep-C1 and strep-C2).

### Oligonucleotide Primer Sequences

Strep-C1 CCG C/GGA CAT CTA CTC C/GGC
Strep-C2 CCG TCC TCC CAG AAG TGC

The two conserved regions chosen spanned DNA encoding 185 amino acids of the predicted Erwinia gene product. In order to bias the primers for use with Streptomyces DNA, which has a high ratio of G and C residues, the amino acid sequence was back translated using G and C residues where possible. This resulted in the oligonucleotides being of mixed sequence where the amino acid could be encoded by more than one DNA sequence.

PCR reactions were performed using the above primers. A number of sets of reactions were performed with varying concentrations of template DNA, primer DNA, dNTPs and magnesium chloride. The denaturing time, annealing temperature and ramp rate of the PCR programme were also varied.

A PCR product of approximately 500 base pairs was seen in some of the reactions using the primers strep-C1 and strep-C2 and the Streptomyces cattleya DNA as template.

The carA to carH gene products may be used to make antibodies for use in identifying in a library streptomycete clones expressing a cross-reacting protein. This is a possible route to the identification of new car genes and new carbapenem antibiotics.

The cloning of the carbapenem biosynthetic genes will now be described in more detail and with reference to the accompanying figures, by way of example only:

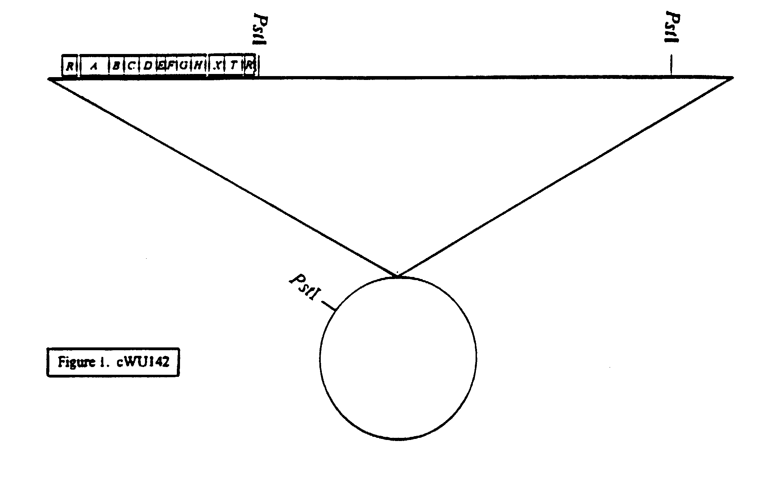

Fig.1 shows schematically the cosmid cWU142, showing the organisation of the car genes R,A,B,C,D,E,F,G,H, as well as unrelated genes (X,T,R') and the three PstI restriction sites. Cosmid cWU142 was obtained by cloning the carbapenem biosynthetic genes into the low copy number plasmid pSF6.

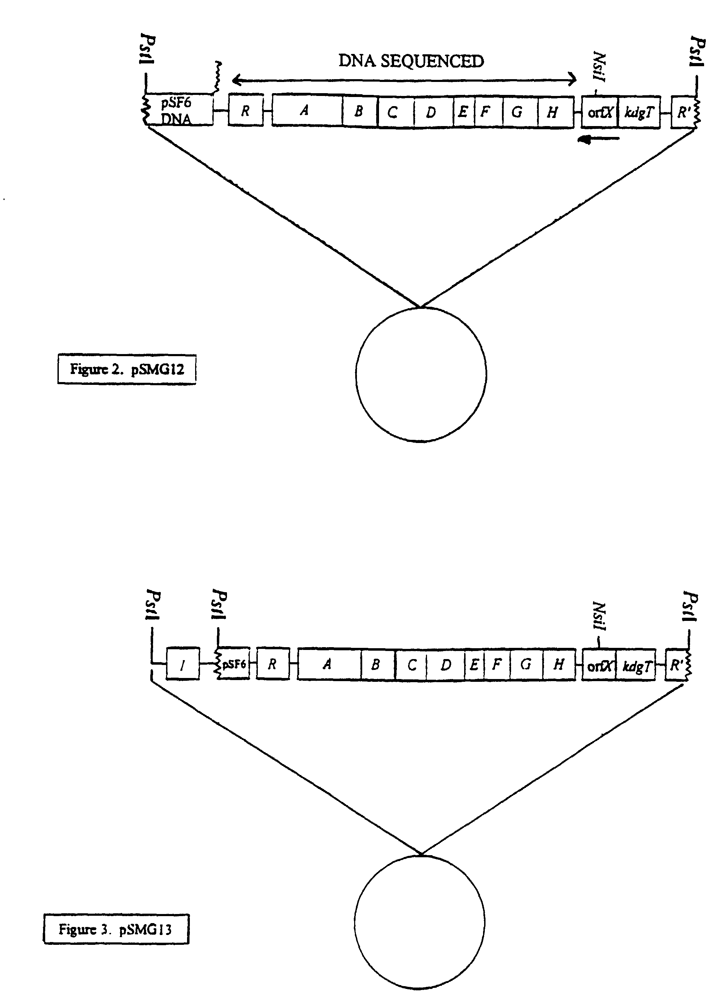

cWU142 was digested with PstI, to yield three large (ie greater than 12kb) fragments. Each of these three fragments was cloned individually in pUC9. The clone that was shown by PCR analysis to contain carR was named, pSMG10. The fragment within this construct was subsequently recloned using PstI in pACYC177 and this construct was named pSMG12 (Fig.2).

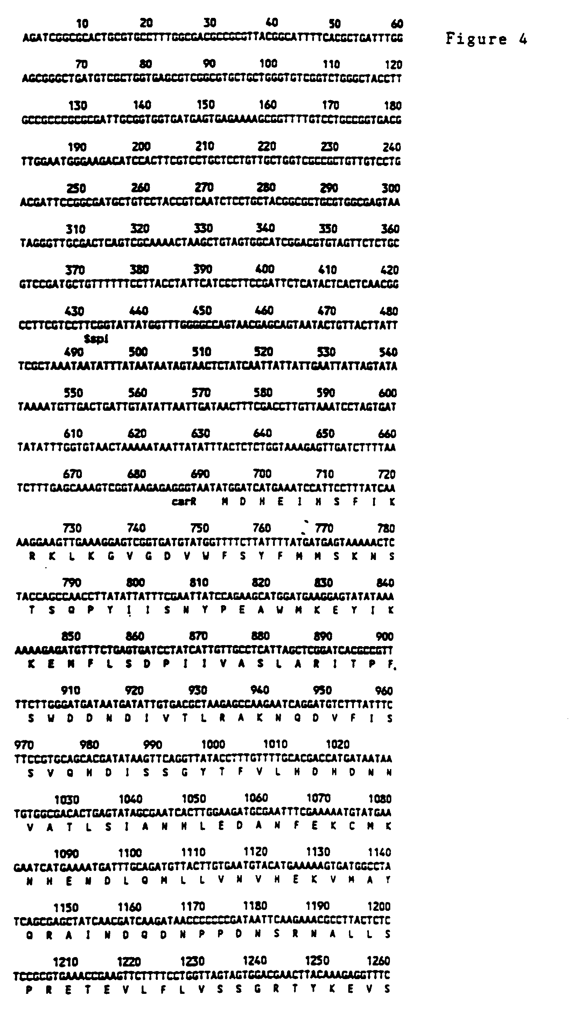

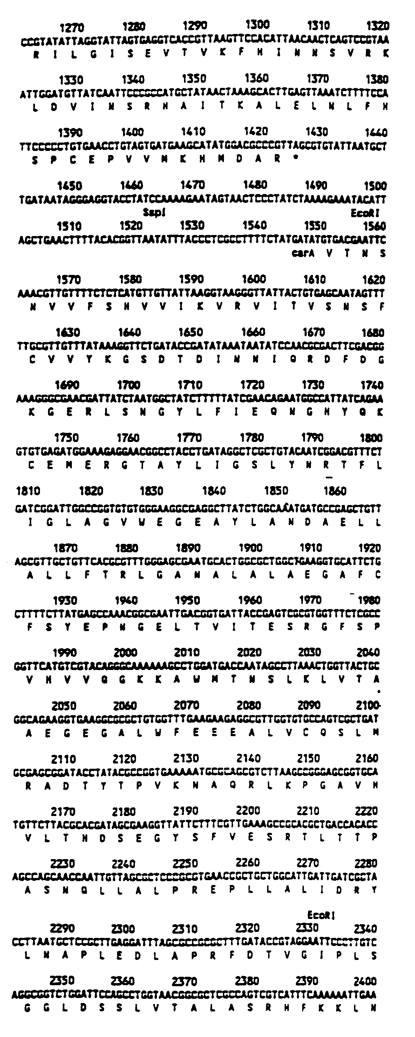

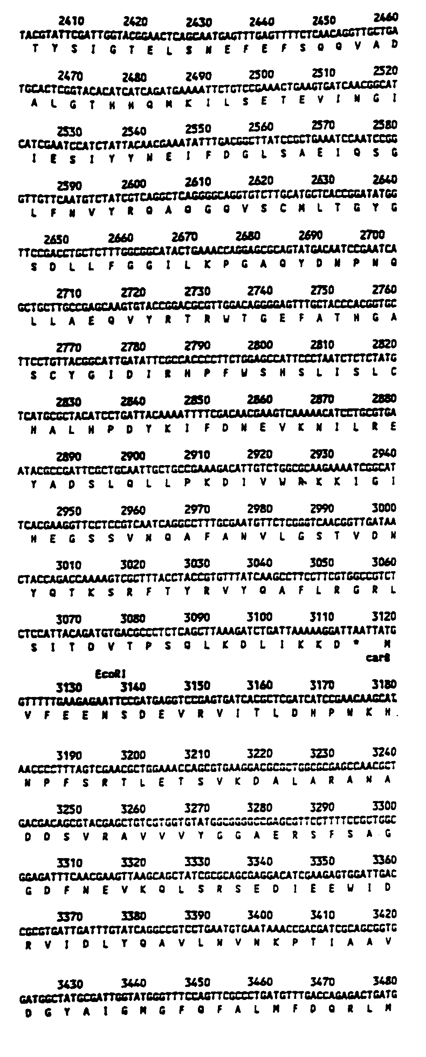

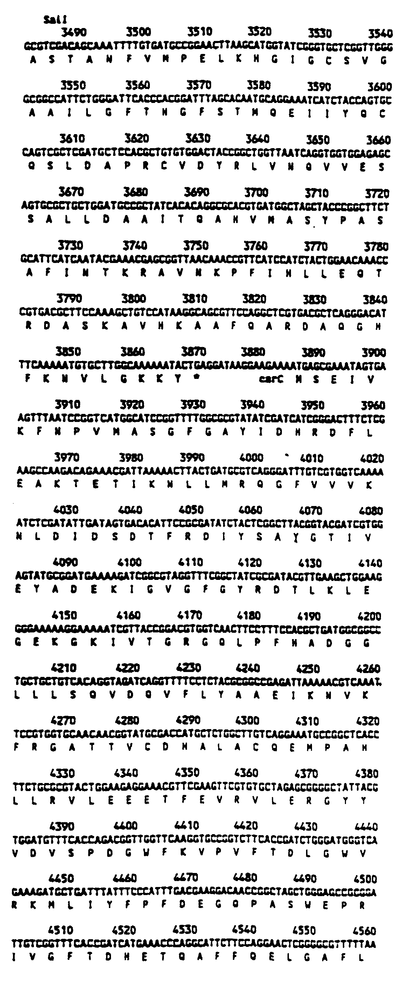

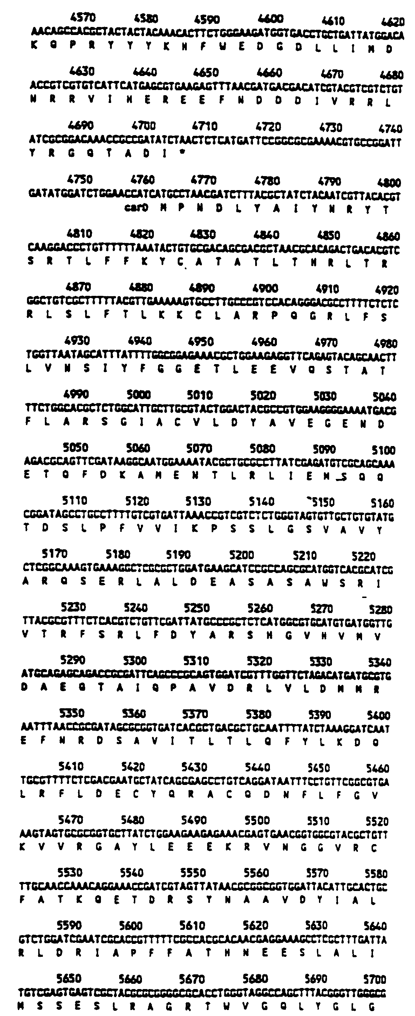

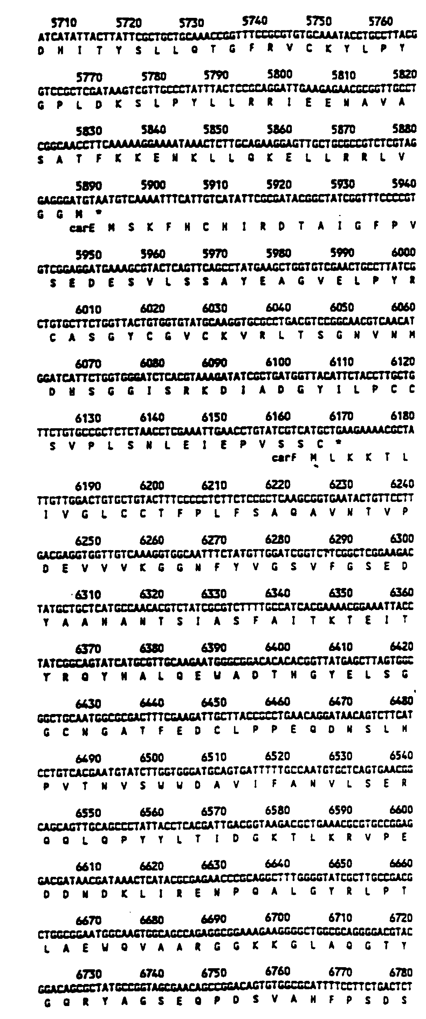

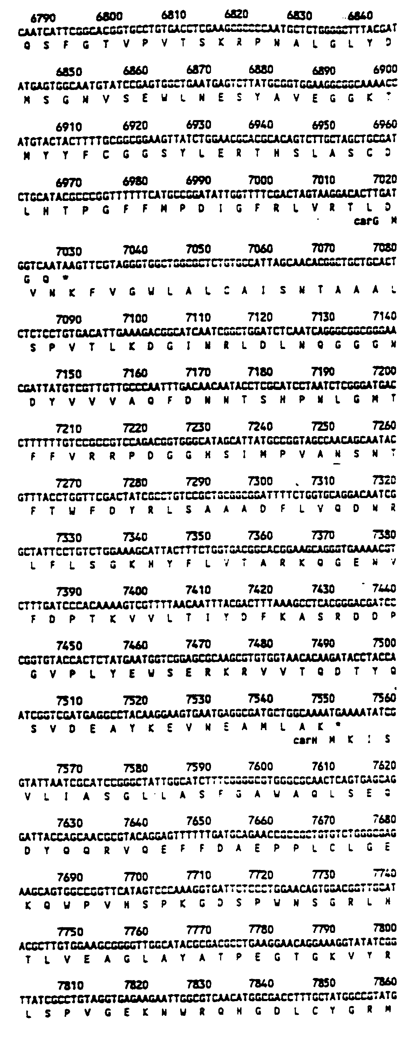

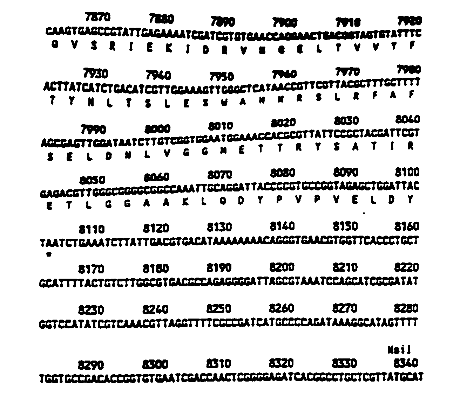

The carI gene had previously been cloned in pSJ100 and been shown to be present on a 2.3kb PstI fragment (S. Jones). This carI containing fragment was cloned into pSMG12 following partial digestion of this plasmid with PstI. The resulting construct was named pSMG13 (Fig.3).

E. coli containing plasmids pSMG12 or pSMG13 was spotted onto a lawn of the carbapenem hyper-sensitive strain of E. coli ("Beecham's E. coli"). A zone of clearing around the pSMG13-containing strain indicated the production by this strain of carbapenem. When exogenous N-(3-oxohexanoyl)-L-homoserine lactone (OHHL) was added to both strains, then the pSMG12 carrying strain could also produce carbapenem.

Fig.4 shows the entire sequence of the carR,carA,carB,carC,carD,carE,carF,carG,carH genes, together with the sequences of the polypeptides that they encode. The sequences of some upstream and downstream DNA are also shown, including a portion of the orfX gene which, like the adjacent kdgT and R¹ -genes do not form part of the carbapenem biosynthetic pathway.

From complementation and cross feeding studies, involving the mutants and cosmids of both Serratia marcescens (Sma) and Erwinia carotovora subspecies cartovora (Ecc) it appeared that all the genes necessary for carbapenem biosynthesis were also contained on 2 Sma cosmids, pNRT1 and pNRT20. These Sma carbapenem cosmids seemed to be functionally interchangeable in many cases with the Ecc cosmid cWU142, and it seemed likely that the putative carbapenem genes might be quite highly conserved between the two genera. Southern blot hybridisations were used to localise the carbapenem gene region on the cosmids and this facilitated the subcloning and sequencing of the Sma carbapenem genes. 8.7kb from the Sma carbapenem gene region was sequenced and was found to contain 9 ORFs, carRABCDEFGH, all transcribed in the same direction, with carABCDEFGH possibly forming an operon. The 9 genes had both similar size and high DNA homology to the 9 genes sequenced from the carbapenem gene region of Ecc. The predicted gene products also showed high amino acid sequence homology with the corresponding gene products from Ecc.

## Claims

1. DNA consisting of:
(a) at least one of the carbapenem synthesis genes carA, carB, carC, carD, carE, carF, carG, carH, as defined in Figure 4;
(b) DNA capable of hybridising to any of the genes defined in (a) and capable of functioning as such genes in the biosynthetic pathway of a carbapenem;
(c) DNA which is equivalent to DNA defined in either (a) or (b) above by virtue of the degeneracy of the genetic code and capable of functioning as such DNA in the carbapenem biosynthetic pathway.

2. DNA consisting of:
(a) the cluster of at least carbapenem synthesis genes carA, carB and carC as defined in Figure 4;
(b) DNA capable of hybridising to the genes defined in (a) and capable of functioning as such genes in the biosynthetic pathway of a carbapenem;
(c) DNA which is equivalent to DNA defined in either (a) or (b) above by virtue of the degeneracy of the genetic code and capable of functioning as such DNA in carbapenem biosynthetic pathway.

3. DNA consisting of:
(a) the cluster of carbapenem synthesis genes carA, carB, carC, carD and carE as defined in Figure 4;
(b) DNA capable of hybridising to the genes defined in (a) and capable of functioning as such genes in the biosynthetic pathway of a carbapenem;
(c) DNA which is equivalent to DNA defined in either (a) or (b) above by virtue of the degeneracy of the genetic code and capable of functioning as such DNA in the carbapenem biosynthetic pathway.

4. DNA according to Claim 1(a), 2(a) or 3(a) respectively, also containing either or both of the regulatory genes carR, as defined in Figure 4, and carI, DNA hybridising thereto in accordance with Claim 1(b), 2(b) or 3(b) respectively, or DNA equivalent thereto in accordance with Claim 1(c), 2(c) or 3(c), respectively.

5. DNA as defined in Claim 1(a), 2(a) or 3(a), wherein the or each gene is a gene of Erwinia carotovora.

6. DNA as defined in Claim 1(b), 2(b) or 3(b), wherein said DNA comprises one or more native genes from a carbapenem encoding organism or partially or wholly synthetic DNA.

7. A polypeptide coded for by DNA as defined in any of Claims 1 to 6.

8. A recombinant DNA molecule including DNA as defined in any of Claims 1 to 6.

9. A recombinant DNA molecule according to Claim 8, including the cluster of genes carA, carB, carC, carD, carE, carF, carG, carH.

10. A recombinant DNA molecule according to Claim 8 or 9 which also contains either or both of the gene carI and carR.

11. A recombinant DNA molecule comprising DNA according to any of claims 2,3,4,9 or 10 which is an expression vector capable of expressing a carbapenem polypeptide coded for by said gene cluster.

12. A recombinant DNA molecule according to Claim 8 which is cosmid cWU142, or either of the plasmids pSMG12 or pSMG13, as defined in Figures 1, 2 and 3, respectively.

13. A method of identifying further DNA coding for production of a carbapenem by using DNA as defined in any of Claims 1 to 5 or a portion of such DNA as a hybridisation probe, incorporating any further DNA so identified into an expression vector, transforming a host with the resulting recombinant expression vector, expressing the polypeptide coded for by said further DNA, and testing said polypeptide for carbapenem production activity.

14. A method according to Claim 13, wherein said further DNA is amplified by means of PCR.

15. A method according to Claim 13 or 14 which is also used to identify genes linked to said further DNA, which genes are involved in carbapenem synthesis.

## Patentansprüche

1. DNS, bestehend aus:
(a) mindestens einem der Gene zur Carbapenem-Synthese carA, carB, carC, carD, carE, carF, carG, carH, wie in Abbildung 4 definiert;
(b) DNS, die zu irgendeinem der in (a) definierten Gene hybridisieren kann und wie diese Gene bei der Biosynthese eines Carbapenems wirken kann,
(c) DNS, die zu der oben entweder in (a) oder (b) definierten DNS aufgrund von Degenerierung des genetischen Codes äquivalent ist und wie diese bei der Carbapenem-Biosynthese wirken kann.

2. DNS, bestehend aus:
(a) dem Cluster aus mindestens den Genen zur Carbapenem-Synthese carA, carB und carC, wie in Abbildung 4 definiert;
(b) DNS, die zu irgendeinem der in (a) definierten Gene hybridisieren kann und wie diese Gene bei der Biosynthese eines Carbapenems wirken kann,
(c) DNS, die zu der oben entweder in (a) oder (b) definierten DNS aufgrund von Degenerierung des genetischen Codes äquivalent ist und wie diese bei der Carbapenem-Biosynthese wirken kann.

3. DNS, bestehend aus:
(a) dem Cluster aus mindestens den Genen zur Carbapenem-Synthese carA, carB, carC, carD und carE, wie in Abbildung 4 definiert;
(b) DNS, die zu irgendeinem der in (a) definierten Gene hybridisieren kann und wie diese Gene bei der Biosynthese eines Carbapenems wirken kann,
(c) DNS, die zu der oben entweder in (a) oder (b) definierten DNS aufgrund von Degenerierung des genetischen Codes äquivalent ist und wie diese bei der Carbapenem-Biosynthese wirken kann.

4. DNS nach Anspruch 1(a), 2(a) oder 3(a), die zusätzlich eines oder beide der regulatorischen Gene carR, wie in Abbildung 4 definiert, oder carl enthält, DNS, die dazu nach Anspruch 1(b), 2(b) oder 3(b), respektive, hybridisiert, oder DNS, die dazu nach Anspruch 1(c), 2(c) oder 3(c), respektive, äquivalent ist.

5. DNS wie in den Ansprüchen 1(a), 2(a) oder 3(a) definiert, wobei das oder jedes Gen ein Gen von Erwinia carotovora ist.

6. DNS wie in den Ansprüchen 1(b), 2(b) oder 3(b) definiert, wobei die besagte DNS ein oder mehrere native Gene aus einen Carbapenem codierenden Organismus oder teilweise oder vollständig synthetische DNS umfaßt.

7. Polypeptid, für das die in irgendeinem der Ansprüche 1 bis 6 definierte DNS codiert.

8. Rekombinantes DNS-Molekül, das die in irgendeinem der Ansprüche 1 bis 6 definierte DNS enthält.

9. Rekombinantes DNS-Molekül nach Anspruch 8, das den Gencluster carA, carB, carC, carD, carE, carF, carG, carH enthält.

10. Rekombinantes DNS-Molekül nach Anspruch 8 oder 9, das zusätzlich eines der oder die beiden Gene carl und carR enthält.

11. Rekombinantes DNS-Molekül nach einem der Ansprüche 2, 3, 4, 9 oder 10, das ein Expressionsvektor ist, der ein Carbapenem-Polypeptid exprimieren kann, für das der besagte Gencluster codiert.

12. Rekombinantes DNS-Molekül nach Anspruch 8, das Cosmid cWU142 oder eines der Plasmiden pSMG12 oder pSMG13 ist, wie in den Abbildungen 1, 2 oder 3, respektive, definiert.

13. Verfahren zur Identifizierung weiterer, für die Produktion von Carbapenem codierender DNS durch Verwendung von DNS, wie in irgendeinem der Ansprüche 1 bis 5 definiert, oder eines Teils solcher DNS als Hybridisierungssonde, wobei jede so identifizierte weitere DNS in einen Exprimierungsvektor eingebaut wird und ein Wirt mit dem resultierenden Exprimierungsvektor transformiert wird, um das durch die besagte weitere DNS codierte Polypeptid zu exprimieren, und durch Prüfung der Aktivität des besagten Polypeptids zur Carbapenemherstellung.

14. Verfahren nach Anspruch 13, wobei die besagte weitere DNS mittels PCR vermehrt wird.

15. Verfahren nach Anspruch 13 oder 14, das zusätzlich zur Identifizierung von Genen, die mit der besagten weiteren DNS verknüpft und an der Carbapenem-synthese beteiligt sind, verwendet wird.

## Revendications

1. ADN constitué
(a) d'au moins l'un des gènes de synthèse des carbapénèmes carA, carB, carC, carD, carE, carF, carG, carH, tels que définis sur la figure 4 ;
(b) d'un ADN capable de s'hybrider à l'un quelconque des gènes définis en (a) et capable de fonctionner en tant que gènes de ce type dans la voie de biosynthèse d'un carbapénème ;
(c) d'un ADN qui est équivalent à l'ADN défini en (a) ou en (b) ci-dessus en vertu de la dégénérescence du code génétique, et capable de fonctionner en tant qu'un ADN de ce type dans la voie de biosynthèse des carbapénèmes.

2. ADN constitué
(a) de la batterie d'au moins les gènes de synthèse des carbapénèmes carA, carB et carC, tels que définis sur la figure 4 ;
(b) d'un ADN capable de s'hybrider à l'un quelconque des gènes définis en (a) et capable de fonctionner en tant que gènes de ce type dans la voie de biosynthèse d'un carbapénème ;
(c) d'un ADN qui est équivalent à l'ADN défini en (a) ou en (b) ci-dessus en vertu de la dégénérescence du code génétique, et capable de fonctionner en tant qu'un ADN de ce type dans la voie de biosynthèse des carbapénèmes.

3. ADN constitué
(a) de la batterie de gènes de synthèse des carbapénèmes carA, carB, carC, carD et CarE, tels que définis sur la figure 4 ;
(b) d'un ADN capable de s'hybrider à l'un quelconque des gènes définis en (a) et capable de fonctionner en tant que gènes de ce type dans la voie de biosynthèse d'un carbapénème ;
(c) d'un ADN qui est équivalent à l'ADN défini en (a) ou en (b) ci-dessus en vertu de la dégénérescence du code génétique, et capable de fonctionner en tant qu'un ADN de ce type dans la voie de biosynthèse des carbapénèmes.

4. ADN, respectivement selon les revendications 1(a), 2(a) ou 3(a), contenant aussi l'un ou l'autre, ou les deux, des gènes régulateurs carR tels que définis sur la figure 4 et carI, un ADN qui s'y hybride, respectivement selon la revendication 1(b), 2(b) ou 3(b), ou un ADN qui leur est équivalent, respectivement selon la revendication 1(c), 2(c) ou 3(c).

5. ADN selon la revendication 1(a), 2(a) ou 3(a), dans lequel le gène ou chaque gène est un gène de Erwinia carotovora.

6. ADN selon la revendication 1(b), 2(b) ou 3(b), dans lequel ledit ADN comprend un ou plusieurs gènes natifs provenant d'un organisme codant les carbapénèmes, ou un ADN partiellement ou entièrement synthétique.

7. Polypeptide codé par un ADN selon l'une quelconque des revendications 1 à 6.

8. Molécules d'ADN recombinant comprenant un ADN selon l'une quelconque des revendications 1 à 6.

9. Molécule d'ADN recombinant selon la revendication 8, qui comprend la batterie de gènes carA, carB, carC, carD, CarE, carF, carG, carH.

10. Molécule d'ADN recombinant selon la revendication 8 ou 9, qui contient aussi le gène carI, ou le gène carR, ou les deux.

11. Molécule d'ADN recombinant comprenant un ADN selon l'une quelconque des revendications 2, 3, 4, 9 ou 10, qui est un vecteur d'expression capable d'exprimer un polypeptide de carbapénème codé par ladite batterie de gènes.

12. Molécule d'ADN recombinant selon la revendication 8, qui est le cosmide cWU142, ou encore le plasmide pSMG12 ou le plasmide pSMG13 ou les deux, tels que définis respectivement sur les figures 1, 2 et 3.

13. Procédé pour identifier un autre ADN codant pour la production d'un carbapénème, par utilisation d'un ADN selon l'une quelconque des revendications 1 à 5 ou d'une portion de cet ADN en tant que sonde d'hybridation, incorporation de tout autre ADN ainsi identifié dans un vecteur d'expression, transformation d'un hôte avec le vecteur d'expression recombinant obtenu, expression du polypeptide codé par ledit autre ADN, et essai dudit polypeptide pour déterminer son activité de production de carbapénèmes.

14. Procédé selon la revendication 11, dans lequel ledit autre ADN est amplifié par une amplification en chaîne par polymérase PCR.

15. Procédé selon la revendication 13 ou 14, qui est aussi utilisée pour identifier des gènes liés audit autre ADN, lesdits gènes étant impliqués dans la synthèse des carbapénèmes.
